# EUROPEAN PATENT APPLICATION

(11) **EP 4 748 431 A1**
(43) Date of publication of application: **27.05.2026**
(21) Application number: 25158337.3
(22) Date of filing: 17.02.2025
(51) Int. Cl.: A61M 39/10, A61M 39/22, F16L 39/02

(54) **CONNECTION SYSTEM FOR CONNECTION TO A TWO-LUMEN CATHETER**

(30) Priority: 25.11.2024 EP 24306954
(71) Applicant: Circum Medtech Pharma, 06200 Nice (FR)
(72) Inventor: TERRIER, Julien, 06200 Nice (FR)
(74) Representative: Icosa

(57) **Abstract**

The present invention relates to a connection system for connecting a two-lumen catheter to an fluid management device, the connection system comprising a catheter connecting device and a management connecting device configured to be connected together, the catheter connecting device comprising a polymeric gasket and a rotor abutted within the housing against the polymeric gasket, the management connecting device being configured to switch between a forward configuration and a reverse configuration by rotation of the socket around the flow axis.

## Description

### FIELD OF INVENTION

The present invention relates to a connection system to fluidically connect two fluidic apparatus.

More precisely, the present invention relates to a connection system to fluidically connect a two-lumen catheter to a fluid management device.

### BACKGROUND OF INVENTION

Connection systems between a catheter and a fluid management device such as a pump or a hemodialysis machine are essential to ensure a safe and reliable interaction between patient and machine, enabling efficient blood flow while minimizing the risk of infection and complications. These systems must guarantee connectivity that is easy for medical staff to handle, while maintaining safety and performance requirements during the hemodialysis procedure.

Several known systems exist but they are not satisfactory.

The mainly used known system is a luer-lock connector. These connectors feature a locking mechanism (usually a twist or click mechanism) to securely attach the catheter to the dialysis machine. However, to reverse the fluidic flow, the connector must be disconnected before being reconnected. This increases the number of manipulations and thus the risk of contamination.

Quick-connect systems are typically used for temporary connections (e.g., in emergency situations or short-term dialysis). These systems allow for fast connection and disconnection of the catheter from the machine, reducing handling time and improving operational efficiency. For example, pressure-fit connectors that snap together with a push are commonly used for their ease of use and rapid setup. However, the risk of accidental disconnection with these systems are high. Moreover, as for luer-lock, to reverse the fluidic flow, the connector must be disconnected before being reconnected thereby increasing the number of manipulation and thus the risk of contamination.

There is thus a need for a reversible connector system allowing for easy connection and disconnection. The connector may also be configured to reduce the risk of accidental disconnection and/or to facilitate and reduce the cost of manufacturing.

### SUMMARY

To this end, the invention relates to a connection system for connecting a two-lumen catheter to a fluid management device, the connection system comprising a catheter connecting device and a management connecting device configured to be connected together,
wherein the catheter connecting device comprises:
   - a housing comprising
      ∘ a first catheter connector and a second catheter connector, each catheter connector being configured to be fluidically connected to a lumen of a two-lumen catheter;
      ∘ a connection aperture opposed to the two catheter connectors;
      ∘ an external surface extending from the catheter connectors to the connection aperture, the catheter connectors and the connection aperture defining a flow axis;
   - a polymeric gasket disposed within the housing, the gasket comprising a proximal gasket surface and a distal gasket surface perpendicular to the flow axis, the gasket comprising two through holes aligned with the catheter connectors, each through hole being in fluidic communication with one of the catheter connectors;
   - a rotor disposed within the housing and abutted against the distal gasket surface, the rotor comprising an opening configured to allow fluidic communication from the catheter connectors towards the connection aperture; and
wherein the management connecting device comprises:
   - a locking element configured to reversibly lock the management connecting device to the catheter connecting device thereby preventing relative movement between the management connecting device and the catheter connecting device along the flow axis;
   - a socket comprising a first device connector configured to be fluidically connected to a fluid management device;
the socket being configured to allow fluidic communication between the first device connector and the opening of the rotor, when the management connecting device and the catheter connecting device are connected together through the connection aperture;
the management connecting device being configured, when the management connecting device and the catheter connecting device are connected, to switch between a forward configuration wherein the first catheter connector is in fluidic communication with the first device connector and a reverse configuration wherein the second catheter connector is in fluidic communication with the first device connector by rotation of the socket around the flow axis.

The rotation of the socket around the flow axis allows leads to a reversible connector to alternatively setting a flow of fluid selectively towards each of the catheter connectors without disconnection.

The locking element allows for easy connection and disconnection.

The use of the polymeric gasket reduces the cost of manufacturing because, contrarily to metal used in known connectors, the surface of the gasket does not have to be polished since the sealing is guaranteed by the elasticity of the polymer even when using a low-polished metallic rotor.

The invention also relates to a connection system for connecting a two-lumen catheter to a fluid management device, the connection system comprising a catheter connecting device and a management connecting device configured to be connected together,
wherein the catheter connecting device comprises:
   - a housing comprising
      ∘ a first catheter connector and a second catheter connector, each catheter connector being configured to be fluidically connected to a lumen of a two-lumen catheter;
      ∘ a connection aperture opposed to the two catheter connectors;
      ∘ an external surface extending from the catheter connectors to the connection aperture, the catheter connectors and the connection aperture defining a flow axis;
   - a polymeric gasket disposed within the housing, the gasket comprising a proximal gasket surface and a distal gasket surface perpendicular to the flow axis, the gasket comprising two through holes aligned with the catheter connectors, each through hole being in fluidic communication with one of the catheter connectors;
   - a rotor disposed within the housing and abutted against the distal gasket surface, the rotor comprising an opening configured to allow fluidic communication from the catheter connectors towards the connection aperture; and
wherein the management connecting device comprises:
   - a locking element configured to reversibly lock the management connecting device to the catheter connecting device thereby preventing relative movement between the management connecting device and the catheter connecting device along the flow axis;
   - a socket comprising a first device connector and a second device connector, each device connector being configured to be fluidically connected to a fluid management device;
the socket being configured to allow fluidic communication between the device connectors and the opening of the rotor, when the management connecting device and the catheter connecting device are connected together through the connection aperture;
the management connecting device being configured, when the management connecting device and the catheter connecting device are connected, to switch between a forward configuration wherein the first catheter connector is in fluidic communication with the first device connector while the second catheter connector is in fluidic communication with the second device connector and a reverse configuration wherein the first catheter connector is in fluidic communication with the second device connector while the second catheter connector is in fluidic communication with the first device connector by rotation of the socket around the flow axis.

According to other advantageous aspect of the invention, the polymeric gasket comprises silicone, thermoplastic elastomer, ethylene propylene diene monomer rubber, and/or mixture thereof.

Advantageously, the thermoplastic elastomer is not harmful for the kidney. Therefore, in case of in the event of detachment of particles from the polymeric gasket during the use, these particles do not agglomerate in the kidney of the user.

According to other advantageous aspect of the invention, the catheter connecting device further comprises a gasket support disposed within the housing, the gasket support comprising a proximal support surface and a distal support surface perpendicular to the flow axis, the gasket support comprising two support channels, each support channel being in fluidic communication with one of the catheter connectors, the proximal gasket surface being abutted against the distal support surface, each through hole being fluidically connected with one of the support channels.

The gasket support advantageously acts as a rigid support for the gasket.

According to other advantageous aspect of the invention, the socket comprises at least one rotational key, the at least one rotational key comprising two socket channels aligned with the device connectors and being configured to cooperate, when the management connecting device and the catheter connecting device are connected, with the opening of the rotor.

This allows to provide rotational locking between the rotor and the socket.

According to other advantageous aspect of the invention, the housing has a general cylindrical shape, the external surface of the housing comprises an annular groove and the locking element comprises at least one socket pin fixed to the socket and configured to slid along the annular groove when the management connecting device and the catheter connecting device are connected thereby allowing rotation of the socket around the flow axis.

This allows to prevent the management connecting device and the catheter connecting device to be detached under low traction

According to other advantageous aspect of the invention, the external surface of the housing comprises a L-shaped groove, the L-shaped groove comprising an insertion part extending parallelly to the flow axis from the connection aperture and a locking part extending perpendicularly to the flow axis from the extremity of the insertion part, the management connecting device further comprising a locking ring configured to surround at least part of the socket, the locking element comprising at least one locking pin protruding inside the locking ring, the at least one locking pin being configured to slide along the L-shaped groove to lock the management connecting device and the catheter connecting device when the at least one locking pin is in the locking part of the L-shaped groove.

The locking ring allows to reduce the risk of accidental disconnection.

According to other advantageous aspect of the invention, the annular groove crosses the insertion part of the L-shaped groove.

Unsing the insertion part for inserting the socket pins avoids to use a force above a predetermined force value to click the socket pins in or out the annular groove.

Moreover, the locking ring surrounding closely the socket pins, this provides an additional security against accidental disconnection because of the flexion of the arms connecting the socket pins to the socket is constrained by the locking ring.

According to other advantageous aspect of the invention, the catheter connecting device further comprises a cap configured to seal the connection aperture when the management connecting device and the catheter connecting device are not connected.

This allows to protect the different elements of the catheter connecting device from contamination and shocks.

According to other advantageous aspect of the invention, the cap comprises at least one cap key, the at least one cap key being configured to cooperate, when the cap seals the connection aperture, with the opening of the rotor thereby preventing rotation of the rotor.

This allows to prevent any undesirable rotation of the rotor and guarantee a predefined orientation of the opening relatively to the through holes.

According to other advantageous aspect of the invention, the catheter connectors are luer-locks.

According to other advantageous aspect of the invention, the catheter connectors comprise a plurality of teeth cooperating with a pin fixed on the housing of the catheter connecting device.

According to other advantageous aspect of the invention, the catheter connecting device comprises biocompatible material.

According to other advantageous aspect of the invention, the connection system further comprises at least one locking haptic element configured to provide haptic feedback on the locking between the management connecting device and the catheter connecting device.

This is advantageous because the connection system may be used by blind or visual deficient people.

According to other advantageous aspect of the invention, further comprising at least one rotational haptic element configured to provide haptic feedback during switching between the forward configuration and the reverse configuration.

This is advantageous because the connection system may be used by blind or visual deficient people.

According to other advantageous aspect of the invention, the two-lumen catheter is a central venous catheter.

According to other advantageous aspect of the invention, the fluid management device is a dialysis device.

According to other advantageous aspect of the invention, the fluid management device is a lock solution injector.

### DEFINITIONS

In the present invention, the following terms have the following meanings:

**"Distal"** refers to a part of an element or an element which is farther from the subject compared to other part of the element or other elements.

**"Fluid"** can be any liquid or gas. For example, fluid can be, but is not limited to, blood, biological fluid, sodium chloride, pharmaceutical fluid lock, saline solution, Intravenous (IV) solution, medication, drugs, anesthetics, urine, nutrient, oxygen, air, pressurized gas, ...

**"Fluidic connection"** refers to a pathway through which a fluid can flow from one place to another.

**"Proximal"** refers to a part of an element or an element which is closer to the subject compared to other part of the element or other elements.

### BRIEF DESCRIPTION OF THE DRAWINGS

**Figure 1** shows an exploded view of the catheter connecting device according to one embodiment of the invention.
**Figure 2** shows another viewing angle of the catheter connecting device of Figure 1.
**Figure 3** shows an exploded view of the management connecting device according **to one** embodiment of the invention comprising two device connectors.
**Figure 4** shows an assembled side view of the catheter connecting device of Figure 1.
**Figure 5** shows another viewing angle of the assembled catheter connecting device of Figure 4.
**Figure 6** shows an assembled view of the management connecting device of Figure 3.
**Figure 7** shows an assembled view of the connection system wherein the catheter connecting device of Figure 1 and the management connecting device of Figure 3 are connected.
**Figure 8** shows sectional view of the assembled connection system of Figure 7.
**Figure 9** shows the catheter connecting device and the management connecting device according to another embodiment of the invention.
**Figure 10** shows an exploded view of the catheter connecting device according to the embodiment of Figure 9.
**Figure 11** shows another viewing angle of the catheter connecting device of Figure 10.
**Figure 12** shows the management connecting device according to one embodiment of the invention comprising a single device connector.
**Figure 13** shows another viewing angle of the management connecting device of Figure 12.
**Figure 14** shows sectional view of the connection system wherein the catheter connecting device of Figure 9 is assembled to the management connecting device of Figure 12.

### DETAILED DESCRIPTION

This invention relates to a connection system 1 for connecting a two-lumen catheter to a fluid management device. The fluid management device may be configured to manage a double flow of biological fluid flowing in the two-lumen catheter. The fluid management device may be configured to manage a single flow of lock solution in the two-lumen catheter. The lock solution may be configured to avoid infection, maintain a good flow rate and keep the catheter clean between the connections. The lock solution may comprise taurolidine, heparin, citrate, sodium chloride, urokinase or any combination thereof. The fluid management device preferably comprises a fluid pump configured to inject or suck fluid in the two-lumen catheter. The fluid management device may be a dialysis device. The two-lumen catheter may be a central venous catheter. The two-lumen catheter may be disposed inside a subject.

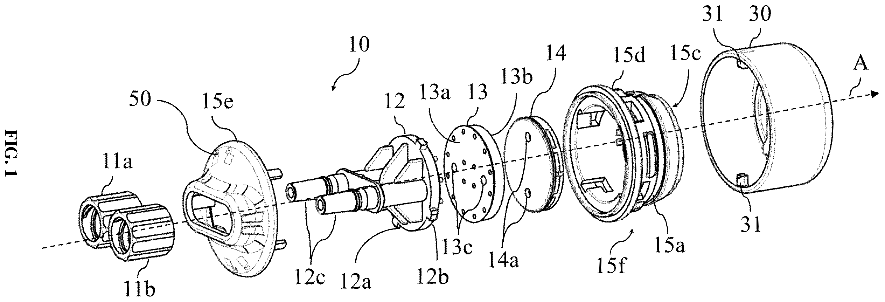

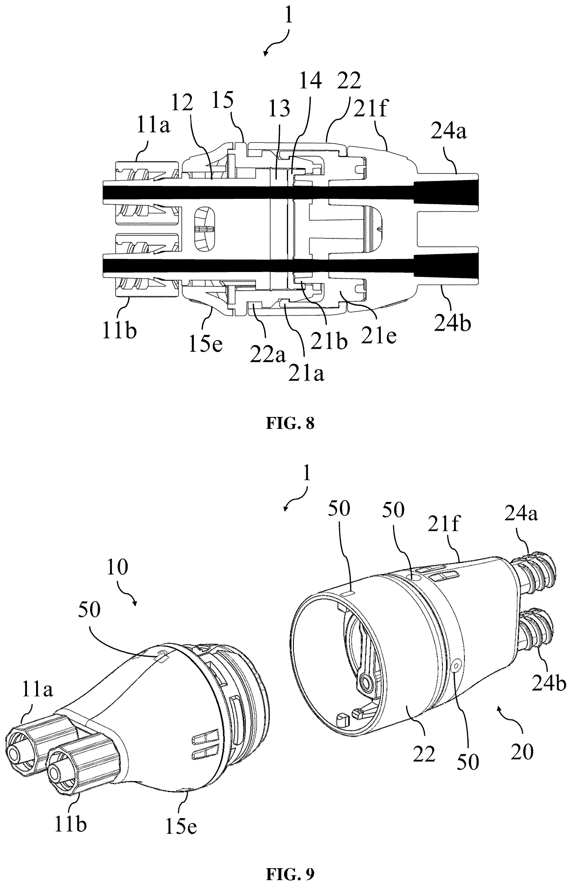

Figures 1-7 and 12-13 represent a specific embodiment of the connection system 1. Figures 9-11 and 14 represent another specific embodiment of the connection system 1. The connection system 1 comprises a catheter connecting device 10 and a management connecting device (20, 40). The catheter connecting device 10 and the management connecting device (20, 40) are configured to be connected together.

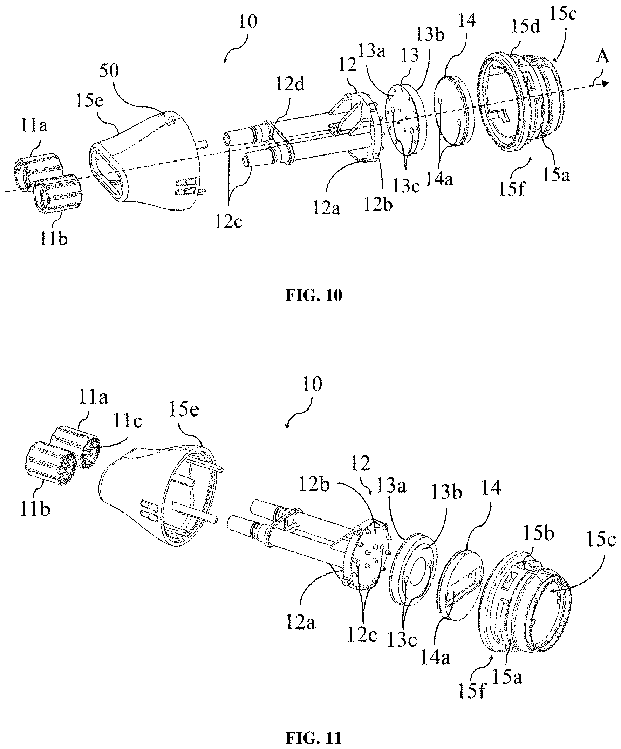

Figure 1 shows an exploded view of the catheter connecting device 10. The catheter connecting device 10 comprises a housing comprising a first catheter connector 11a and a second catheter connector 11b. Each catheter connector (11a, 11b) is configured to be fluidically connected to a lumen of a two-lumen catheter. For example, the first catheter connector 11a is used to transport a fluid (for example blood) out of the subject, i.e. from the subject to the fluid management device, while the second catheter connector 11b is used to transport a fluid into the subject, i.e. the fluid management device from to the subject. The first catheter connector 11a and the second catheter connector 11b may have different colors so as to easily determine the flowing direction of the fluid. For example, the first catheter connector 11a used to transport the fluid out of the subject is red while the second catheter connector 11b used to transport the fluid into the subject is blue thereby corresponding to the representation of the blood circulation. The catheter connectors (11a, 11b) may be luer locks. As represented in Figure 11, the catheter connectors (11a, 11b) may comprise a plurality of teeth 11c cooperating with a pin 12d fixed on the housing of the catheter connecting device 10 as represented in Figure 10. This is advantageous because luer locks have a large screw thread which is easily reversible and which may thus lead to disconnection of the connectors. The teeth 11c increase the resistance of the connectors against the rotation by abutting against the pin 12d, thus avoiding accidental disconnection. The presence of the teeth 11c and the pin 12d is compatible with the embodiment of figures 1-7.

The housing further comprises a connection aperture 15c opposed to the two catheter connectors (11a, 11b).

The catheter connectors (11a, 11b) and the connection aperture 15c being oppositely disposed, they define a flow axis A corresponding to a general axis around which the housing extends.

The housing further comprises a wall extending from the catheter connectors (11a, 11b) to the connection aperture 15c in the direction of the flow axis A. The housing may have any shape. Preferably, the housing has a general cylindrical shape extending around the flow axis A. The housing may be a single piece. In Figures 1 and 10, the wall comprises two pieces: a proximal wall 15e and a distal wall 15f detachable from each other to ease the change of the elements comprised in the housing. The catheter connectors (11a, 11b) may be detachable from the wall. In figure 1, the proximal wall 15e has a short length, lower than 20 mm, measured along the flow axis A. This allows to minimize the total volume of fluid contained in the connection system 1 which is advantageous especially for the injection of lock solutions which are very expensive. In figure 10, the proximal wall 15e has a longer length measured along the flow axis A compared to figure 1. For example, the length of the proximal wall 15e is ranging from 20 mm to 50 mm, preferably from 35 mm to 30 mm. This advantageously allows to avoid any contact between the fingers of the user and the connectors during the connection and disconnection of the connection system 1. To maintain the total volume of fluid contained in the connection system 1 as small as possible when injecting lock solutions, the catheter connecting device 10 may be connected to an embodiment of the management connecting device 40 which may be used specially for injection of lock solutions as explained hereafter. In figure 10, the proximal wall 15e has a distal open extremity configured to surround the gasket support 12. Preferably, the distal open extremity of the proximal wall 15e is circular. In figure 10, the proximal wall 15e has a proximal open extremity configured to surround the support channels 12c. Preferably, the proximal open extremity of the proximal wall 15e is a rounded rectangle or an oval. The size of the distal open extremity is preferably larger than the size of the proximal open extremity.

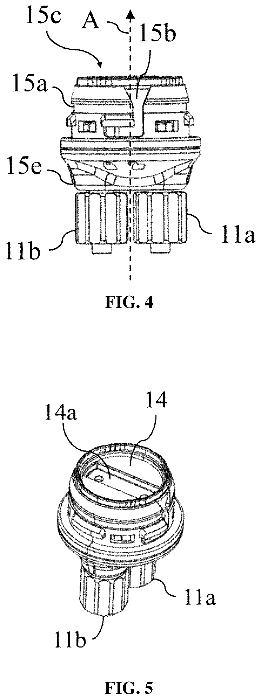

The housing allows to protect the different elements of the catheter connecting device 10 from contamination and shocks as represented in Figure 5 wherein the elements are disposed within the housing.

The catheter connecting device 10 further comprises a gasket 13 and a rotor 14 both disposed within the housing.

The gasket 13 is a polymeric gasket. Therefore, the gasket 13 is in polymer. The polymer may be chosen among, but not limited to: silicone, thermoplastic elastomer (TPE), ethylene propylene diene monomer (EPDM) rubber, or a mixture thereof. Thermoplastic elastomer (TPE) and ethylene propylene diene monomer (EPDM) rubber are advantageous compared to silicone because they are less damaged by repetitive friction with the rotor and they are not causing liver problems when resulting polymer microparticles are flowing in the blood flow of the user.

The gasket 13 comprises a proximal gasket surface 13a and a distal gasket surface 13b. The proximal gasket surface 13a and the distal gasket surface 13b are preferably perpendicular to the flow axis A. The gasket 13 comprising two through holes 13c aligned with the catheter connectors (11a, 11b). Each through hole 13c allows a fluidic communication with one of the catheter connectors (11a, 11b).

In Figure 1, the catheter connecting device 10 further comprises a gasket support 12 disposed within the housing. The gasket support 12 acts as a rigid support for the gasket 13. The gasket 13 is preferably abutted against the gasket support 12. More precisely, the gasket support 12 comprises a proximal support surface 12a and a distal support surface 12b. The proximal support surface 12a and a distal support surface 12b are preferably perpendicular to the flow axis A. The proximal gasket surface 13a is abutted against the distal support surface 12b.

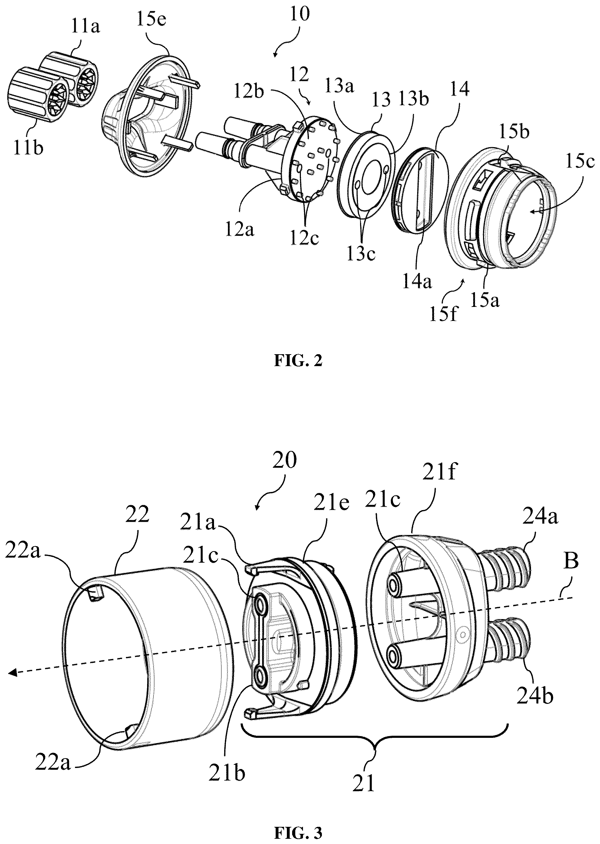

Moreover, the gasket support 12 also acts as a conduit fluidically connecting the through holes 13c to the catheter connectors (11a, 11b). To do so, the gasket support 12 comprises two support channels 12c aligned with the catheter connectors (11a, 11b) and the gasket 13 is abutted against the gasket support 12 so that the through holes 13c are aligned with the support channels 12c as better viewed in Figure 2. The support channels 12c may form a projection outside the proximal support surface 12a as in Figure 1. A funnel shape of the proximal part of the wall of the housing as represented in Figure 1 is advantageous because the projection of the support channels 12c outside the proximal support surface 12a may cooperate with the funnel shape to avoid rotation between the support channels 12c and the catheter connectors (11a, 11b) fixed to the extremity of the funnel shape.

To maintain the alignment between the through holes 13c and the support channels 12c, the catheter connecting device 10 may comprise a plurality of pits disposed in the proximal gasket surface 13a cooperating with protrusions protruding from the distal support surface 12b as better shown in Figure 2.

The rotor 14 is abutted against the distal gasket surface 13b. More precisely, the rotor 14 comprises a proximal rotor surface and a distal rotor surface. The proximal rotor surface and the distal rotor surface are preferably perpendicular to the flow axis A. The proximal rotor surface is abutted against the distal gasket surface 13b.

The rotor 14 is rotationally disposed in the housing therefore allowing the rotation of the rotor 14 around the flow axis 1 relatively to the gasket 13. The rotor 14 is preferably metallic to present a better resistance to the rotational forces.

The use of the polymer in the gasket 13 is advantageous because, contrarily to metal used in known connectors, the surface of the gasket 13 does not have to be polished since the sealing is guaranteed by the elasticity of the polymer even when using a low-polished metallic rotor. However, in another embodiment, the gasket 13 may be a metallic gasket while the rotor 14 is a polymeric rotor. In another embodiment, both the gasket 13 and the rotor 14 are polymeric.

The rotor 14 comprises an opening 14a configured to allow fluidic communication from the catheter connectors (11a, 11b) towards the connection aperture 15c. In Figure 1, the opening 14a comprises two holes opening at the proximal rotor surface, the two holes communicating with a unique merged hole opening at the distal rotor surface as better viewed in Figure 2.

The catheter connecting device 10 may be configured to stay connected on the two-lumen catheter connected to a subject during several days or weeks. Therefore, to increase the comfort of a subject carrying the two-lumen catheter and the catheter connecting device 10, the material of the catheter connecting device 10 is preferably chosen to lead to a low-weight catheter connecting device 10. For example, the weight of the catheter connecting device 10 is ranging from 5 g to 50 g, preferably from 10 g to 20 g. For example, material of at least some of elements of the catheter connecting device 10 may be polyethylene terephthalate glycol (PETG), Polyphenylsulfone (PPSU), thermoplastic elastomer, silicone, ethylene propylene diene monomer (EPDM) rubber or mixture thereof (e.g. materials of elements of the catheter connecting device 10 may be distinct polymers).

Moreover, the connection system 1 is preferably configured to avoid any contamination inside the catheter. For example, the catheter connecting device 10 comprises biocompatible material. In another example compatible with the previous example, the catheter connecting device 10 comprises a cap 30 represented in Figure 1 and configured to seal the connection aperture 15c. The cap 30 may be connected to and disconnected from the housing. The cap 30 can be connected to the housing when the catheter connecting device 10 and the management connecting device 20 are not connected together.

Due to the possible rotation of the rotor 14 around the flow axis A, the opening 14a is not necessarily aligned with the through holes 13c. To prevent any undesirable rotation of the rotor 14 and guarantee a predefined orientation of the opening 14a relatively to the through holes 13c, the cap 30 may comprise at least one cap key configured to cooperate, when the cap 30 seals the connection aperture 15c, with the opening 14a of the rotor 14 thereby preventing rotation of the rotor 14.

The management connecting device (20, 40) represented in Figures 3, 9 and 12 comprises a socket (21, 41) comprising a first device connector (24a, 44). The first device connector (24a, 44) is configured to be fluidically connected to a fluid management device. For example, the first device connector (24a, 44) is configured to transport a fluid (for example blood) towards the fluid management device or to carry a fluid (for example sodium chloride, pharmaceutical lock solution for catheter) from the fluid management device.

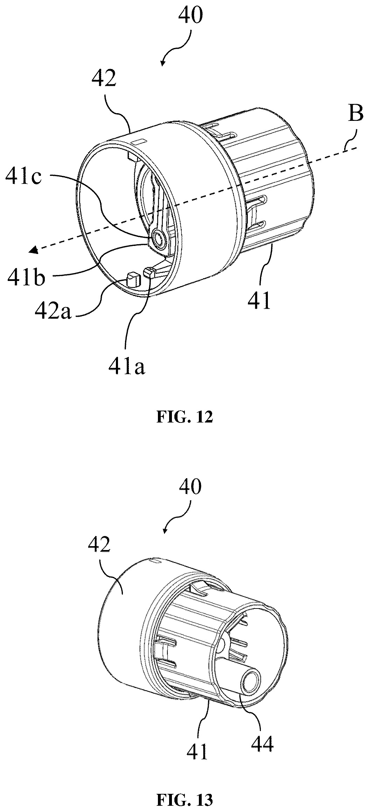

Contrarily to the management connecting device 40 represented in Figure 12, the management connecting device 20 represented in Figures 3 and 9 further comprises a second device connector 24b. The second device connector 24b is configured to be fluidically connected to a fluid management device. For example, the second device connector 24b is used to carry a fluid (for example blood or a pharmaceutical lock solution) from the fluid management device.

The socket (21, 41) allows fluidic communication between the device connectors (24a, 24b, 44) and the opening 14a of the rotor 14 and thus, when the opening 14a of the rotor 14 is aligned with the through holes 13c, fluidic communication occurs between the device connectors (24a, 24b, 44) and the catheter connectors (11a, 11b). The management connecting device 40 represented in Figure 12 comprises only the first device connector 44. It therefore allows to connect only one of the catheter connectors (11a, 11b) with a single lumen fluid management device such as a syringe. This is advantageous for injection of lock solutions or for blood sampling for example. The management connecting device 20 represented in Figures 1-7 and 9-10 comprises two device connectors (24a, 24b). It therefore allows to connect the two catheter connectors (11a, 11b) with a two-lumen fluid management device. This is advantageous for dialysis for example.

To do so, the management connecting device (20, 40) is configured to be connected to the catheter connecting device 10. Advantageously, the management connecting device 40 represented in Figure 12 and the management connecting device 20 represented in Figures 1-7 and 9-10 have the same proximal structure. To connect the management connecting device (20, 40) to the catheter connecting device 10, the extremities of the device connectors (24a, 24b, 44) to be connected to the fluid management device are facing away the extremities of the catheter connectors (11a, 11b) to be connected to the two-lumen catheter. In other words, the extremities of the device connectors (24a, 24b, 44) opposed to the fluid management device are facing towards the rotor 14.

When the management connecting device (20, 40) is connected to the catheter connecting device 10, the device connectors (24a, 24b, 44) are aligned with the opening 14a of the rotor 14. The device connectors (24a, 24b, 44) preferably extend as socket channels (21c, 41c) along a connection axis B so that, when the management connecting device (20, 40) is connected to the catheter connecting device 10, the socket channels (21c, 41c) abut against the rotor 14 to provide sealing between the management connecting device (20, 40) and the catheter connecting device 10. This advantageously allows to prevent leaks, air penetration and/or contamination. The management connecting device (20, 40) and the catheter connecting device 10 are thus connected together through the connection aperture 15c, the socket channels (21c, 41c) passing through the connection aperture 15c. When the management connecting device (20, 40) is connected to the catheter connecting device 10, the connection axis B is parallel to the flow axis A.

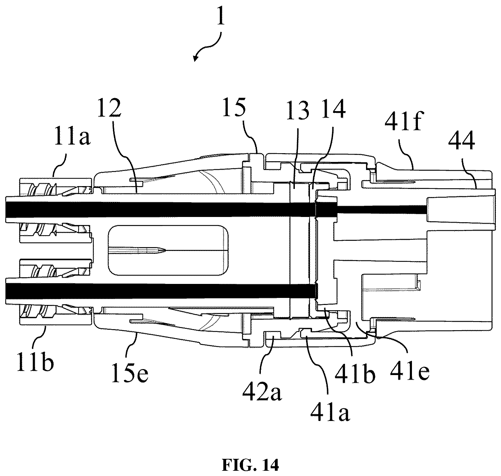

When the management connecting device (20, 40) and the catheter connecting device 10 are connected, the management connecting device (20, 40) has a forward configuration wherein the first catheter connector 11a is in fluidic communication with the first device connector (24a, 44). The forward configuration is illustrated in Figures 8 and 14 wherein the fluid is shown in black. In the forward configuration, the second device connector 24b of the management connecting device 20 represented in Figures 3 and 9 is in fluidic communication with the second catheter connector 11b. In the forward configuration, the management connecting device 40 represented in Figure 9 closes the fluid communication of the second catheter connector 11b.

The management connecting device (20, 40) also has a reverse configuration wherein the second catheter connector 11b is in fluidic communication with the first device connector (24a, 44). In the reverse configuration, the second device connector 24b of the management connecting device 20 represented in Figures 3 and 9 is in fluidic communication with the first catheter connector 11a. In the reverse configuration, the management connecting device 40 represented in Figure 9 closes the fluid communication of the first catheter connector 11a.

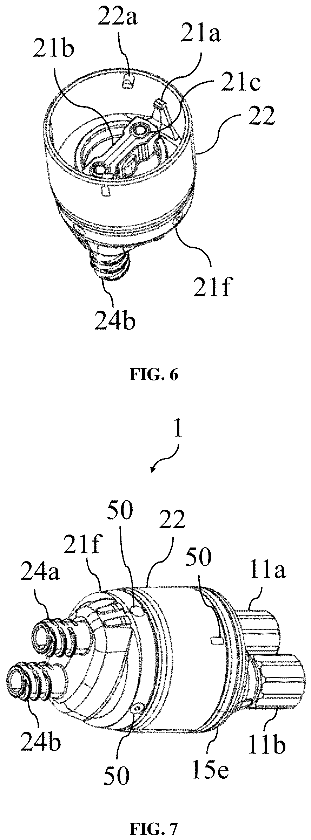

The management connecting device (20, 40) is configured to switch between the forward configuration and the reverse configuration by rotation of the socket (21, 41) around the flow axis A. To do so, the rotor 14 is rotationally locked to the socket (21, 41) when the management connecting device (20, 40) and the catheter connecting device 10 are connected. Figure 7 shows the management connecting device (20, 40) during the switch phase when the catheter connectors (11a, 11b) and the apparatus connectors (24a, 24b, 44) are not aligned.

The opening 14a of the rotor 14 represented in Figures 1 and 2 is advantageous. Indeed, the merged hole opening at the distal rotor surface allows the insertion of the socket channels (21c, 41c) of the socket (21, 41) in the merged hole and a rotational locking between the socket channels (21c, 41c) (and thus the device connectors (24a, 24b, 44)) and the opening 14a of the rotor 14. The socket channels (21c, 41c) thus act as rotational keys. Therefore, rotating the socket (21, 41) around the flow axis A implies a rotation of the rotor 14 and thus a rotation of the opening 14a so that the two holes opening at the proximal rotor surface are selectively in fluidic communication with the through holes 13c.

The socket (21, 41) may comprise two pieces as represented in figure 3 a proximal part (21e, 41e) and a distal part (21f, 41f). The device connectors (24a, 24b, 44) may be fixed on the distal part (21f, 41f). In figure 3, the distal part 21f has a short length measured along the flow axis A. This allows to minimize the total volume of fluid contained in the connection system 1 which is advantageous especially for the injection of lock solutions which are very expensive. In figure 9, the distal part 21f has a longer length measured along the flow axis A compared to figure 3. This advantageously allows to avoid any contact between the fingers of the user and the connectors during the connection and disconnection of the connection system 1. In figure 9, the distal part 21f has a distal open extremity configured to surround the proximal part 21e. Preferably, the proximal open extremity of the distal part 21f is circular. In figure 9, the distal part 21f has a distal open extremity configured to surround the device connectors (24a, 24b). Preferably, the distal extremity of the distal part 21f is a rounded rectangle or an oval. The size of the proximal open extremity is preferably larger than the size of the distal extremity.

The proximal part (21e, 41e) may comprise a rotational key (21b, 41b). The shape of the rotational key (21b, 41b) preferably corresponds to the shape of the opening 14a of the rotor 14 to rotationally lock the rotor 14 to the proximal part (21e, 41e) when the management connecting device 20 and the catheter connecting device 10 are connected. The rotational key (21b, 41b) comprises part of the two socket channels (21c, 41c).

The distal part (21f, 41f) and proximal part (21e, 41e) are rotationally fixed together with an alignment between the two parts of the socket channels (21c, 41c).

The management connecting device (20, 40) comprises a locking element configured to reversibly lock the management connecting device (20, 40) to the catheter connecting device 10 thereby preventing relative movement between the management connecting device (20, 40) and the catheter connecting device 10 along the flow axis A.

In Figures 1-7 and 9-13, the locking element is fixed on the management connecting device (20, 40) and cooperates with the housing, for example, with an external surface 15d of the wall of the housing.

In one example, the external surface 15d of the housing comprises an annular groove 15a as represented in Figures 1, 2, 4, 5, 10 and 11 and the locking element comprises socket pins (21a, 41a) fixed to the socket (21, 41) as represented in Figures 3, 6 and 12. The socket pins (21a, 41a) are configured to slid along the annular groove 15a when the management connecting device (20, 40) and the catheter connecting device 10 are connected thereby allowing rotation of the socket (21, 41) around the flow axis A at a 360° angle to allows the switching between the forward configuration and the reverse configuration. The socket pins (21a, 41a) cooperating with the annular groove 15a allows to prevent the management connecting device (20, 40) and the catheter connecting device 10 to be detached under low traction. The socket pins (21a, 41a) may be fixed to the socket (21, 41) with arms extending parallelly to the flow axis A. The arms fixing the socket pins (21a, 41a) to the socket (21, 41) may have a small degree of flexibility to allow attachment or detachment by excreting a force parallel to the flow axis A above a predetermined force value.

In another embodiment of locking between the management connecting device (20, 40) and the catheter connecting device 10, the external surface 15d of the housing comprises a L-shaped groove 15b. As better viewed in Figure 4, the L-shaped groove 15b comprises an insertion part extending parallelly to the flow axis A from the connection aperture 15c and a locking part extending perpendicularly to the flow axis A from the extremity of the insertion part. In this embodiment, the management connecting device (20, 40) further comprises a locking ring (22, 42) configured to surround at least part of the socket 21. In Figures 6, 7 and 12, the proximal part (21e, 41e) of the socket (21, 41) is surrounded by the locking ring (22, 42) while the distal part (21f, 41f) is disposed beside the locking ring (22, 42). The locking element comprises locking pins (22a, 42a) protruding inside the locking ring (22, 42). To lock the management connecting device (20, 40) to the catheter connecting device 10, the locking pins (22a, 42a) are slid along the insertion part of the L-shaped groove 15b. The management connecting device (20, 40) thus move along the flow axis A to connect to the catheter connecting device 10. When the management connecting device (20, 40) and the catheter connecting device 10 are connected, i.e., when the socket channels (21c, 41c) abut against the rotor 14, the locking ring (22, 42) is rotated around the fluid axis A so that the pins 22a slid along the locking part of the L-shaped groove 15b thereby providing a locking between the management connecting device (20, 40) and the catheter connecting device 10. The locking ring (22, 42) is preferably free in rotation about the socket (21, 41) so that, when the locking ring (22, 42) locks the management connecting device (20, 40) and the catheter connecting device 10, the socket (21, 41) is able to rotate freely to switch between the forward configuration and the reverse configuration.

In the embodiment represented in the figures, the connection system 1 comprises the two above-mentioned embodiments for locking the management connecting device (20, 40) and the catheter connecting device 10. The housing comprises the annular groove 15a and the L-shaped groove 15b while the management connecting device (20, 40) comprises the corresponding socket pins (21a, 41a) fixed to the socket (21, 41) and locking pins (22a, 42a) protruding inside the locking ring (22, 42). Advantageously, the annular groove 15a crosses the insertion part of the L-shaped groove 15b. Therefore, the socket pins 21a inserted in the annular groove 15a by first sliding the socket pins 21a along part of the insertion part of the L-shaped groove 15b following the locking pins (22a, 42a) and then rotating the socket (21, 41) so that the socket pins (21a, 41a) slid along the annular groove 15a. This thus avoids to use a force above a predetermined force value to click the socket pins (21a, 41a) in or out the annular groove 15a. Moreover, the locking ring (22, 22) surrounding closely the socket pins (21a, 41a), this provides an additional security against accidental disconnection because of the flexion of the arms connecting the socket pins (21a, 41a) to the socket (21, 41) is constrained by the locking ring (22, 42). Preferably, the connection system 1 is configured so that a traction force may be as large as 35 N without accidental disconnection.

The presence of the annular groove 15a or the L-shaped groove 15b is also advantageous when the catheter connecting device 10 comprises a cap 30. Indeed, the cap 30 may comprise at least one cap pin 31 protruding inside the cap 30 as represented in Figure 1. When the management connecting device (20, 40) and the catheter connecting device 10 are not connected, the cap 30 may be attached and locked to the catheter connecting device 10 by using the same annular groove 15a or L-shaped groove 15b.

Other embodiments of locking element to reversibly lock the management connecting device (20, 40) to the catheter connecting device 10 preventing relative movement between the management connecting device (20, 40) and the catheter connecting device 10 along the flow axis A are possible according to the need.

The connection system 1 may advantageously further comprise at least one locking haptic element configured to provide haptic feedback to the patient and/or medical staff on the locking between the management connecting device (20, 40) and the catheter connecting device 10. Moreover, the connection system 1 may comprise at least one rotational haptic element configured to provide haptic feedback during switching between the forward configuration and the reverse configuration. This is advantageous because the connection system 1 may be used by blind or visual deficient people.

The connection system 1 may also comprise at least one visual indicator 50 to indicate to the user in which of the forward of reverse configuration the connection system 1 is and/or if the management connecting device (20, 40) and the catheter connecting device 10 are locked.

Figures 1 and 10 shown an example of indicators 50 for the locking disposed on the external surface of the housing.

Figures 7 and 8 shows an example of indicators 50 for the configuration disposed on the socket 21 and an indicator 50 for the locking disposed on the locking ring 22.

The size of the connection system 1 measured perpendicularly to the flow axis A may range from 10 mm to 50 mm, preferably from 25 mm to 35 mm.

The size of the catheter connecting device 10 measured parallelly to the flow axis A may range from 10 mm to 70 mm, preferably from 20 mm to 60 mm.

The size of the management connecting device 20 measured parallelly to the flow axis A may range from 10 mm to 70 mm, preferably from 30 mm to 60 mm.

The size of the management connecting device 40 measured parallelly to the flow axis A may range from 10 mm to 60 mm, preferably from 20 mm to 40 mm.

Internal diameter of a catheter connector (11a, 11b) may range from 0.5 mm to 5 mm, preferably from 2 mm to 3.2 mm. Diameters of catheter connectors (11a, 11b) may be equal. Alternatively, diameters of catheter connectors (11a, 11b) may be different.

Internal diameter of a device connector (24a, 24b, 44) may range from 0.5 mm to 5 mm, preferably from 2 mm to 3.2 mm. The diameter of the first device connector 24a may be equal to the diameter of the second device connector 24b. Alternatively, diameters of the two device connectors (24a, 24b) may be different.

The volume of the fluidic connections of the connection system 1 may range from 0.1 mL to 2 mL, preferably from 0.2 mL to 0.8 mL.

These sizes provide a connection system 1 which allows an extracorporeal circulation at a maximum blood flow comprised between 250 mL/min and 600 mL/min and a pressure ranging from -450mmHg to 750mmHg.

The invention also relates to an assembling method for assembling the connection system 1. The assembling method comprises connecting the management connecting device (20, 40) to the catheter connecting device 10 by abutting the socket (21c, 41c) against the rotor 14 and locking the management connecting device (20, 40) to the catheter connecting device 10 using the locking element.

In the embodiment wherein the external surface 15d of the housing comprises an annular groove 15a and the locking element comprises socket pins (21a, 41a) fixed to the socket (21, 41), the locking is performed by applying a pressure on the management connecting device (20, 40) parallel to the flow axis A in the direction of the catheter connectors (11a, 11b) so that the socket pins (21a, 41a) settle in the annular groove 15a.

In the embodiment wherein the external surface 15d of the housing comprises a L-shaped groove 15b and the locking element comprises locking pins (22a, 42a), the locking is performed by sliding the locking pins (22a, 42a) along the insertion part of the L-shaped groove 15b from the connection aperture 15c by moving the management connecting device (20, 40) along the flow axis A and then rotating the locking ring (22, 42) around the fluid axis A so that the pins (22a, 42a) slid along the locking part of the L-shaped groove 15b.

In the embodiment wherein the housing comprises the annular groove 15a and the L-shaped groove 15b while the management connecting device (20, 40) comprises the corresponding socket pins (21a, 41a) fixed to the socket (21, 41) and locking pins (22a, 42a) protruding inside the locking ring (22, 42), the locking is performed by:
- sliding the locking pins (22a, 42a) along the insertion part of the L-shaped groove 15b from the connection aperture 15c by moving the management connecting device (20, 40) along the flow axis A;
- sliding the socket pins (21a, 41a) along the insertion part of the L-shaped groove 15b from the connection aperture 15c by continuing the movement of the management connecting device (20, 40) along the flow axis A;
- rotating the locking ring (22, 42) so that the locking pins (22a, 42a) slid along the locking part of the L-shaped groove 15b;
- rotating the socket (21, 41) so that the socket pins (21a, 41a) slid along the annular groove 15a.

The invention also relates to a switching method for switching the connection system 1 from the forward configuration to the reverse configuration, the method comprising performing the assembling method of the connection system 1 as described above and rotating the device connectors (24a, 24b, 44) by 180° around the flow axis A relatively to the catheter connectors (11a, 11b).

Performing the switching method with the fluid management device 20 comprising two device connectors (24a, 24b) leads to the first catheter connector 11a passing from a fluidic communication with the first device connector 24a to a fluidic communication with the second device connector 24b while the second catheter connector 11b is passing from a fluidic communication with the second device connector 24b to a fluidic communication with the first device connector 24a and vice-versa.

Performing the switching method with the fluid management device 40 comprising a single device connector 44 leads to the device connector 44 passing from a fluidic communication with the first catheter connector 11a while the second catheter connector 11b is closed to a fluidic communication with the second catheter connector 11b while the first catheter connector 11a is closed and vice-versa.

The invention also relates to a connecting method for connecting the connection system 1 to a two-lumen catheter and a two-lumen fluid management device. The connecting method comprises:
- fluidically connecting each catheter connector (11a, 11b) to a lumen of the two-lumen catheter;
- fluidically connecting each device connector (24a, 24b) of the management connecting device 20 comprising two device connectors to a lumen of the fluid management device;
- performing the assembling method for assembling the connection system 1 as described above.

These three steps can be performed in any order. For example, the assembly of the catheter can be performed before the fluidic connection to the two-lumen catheter or the fluid management device.

The invention also relates to a connecting method for connecting the connection system 1 to a two-lumen catheter and a single lumen fluid management device. The connecting method comprises:
- fluidically connecting each catheter connector (11a, 11b) to a lumen of the two-lumen catheter;
- fluidically connecting the device connector 44 of the management connecting device 40 comprising a single device connector to the fluid management device;
- performing the assembling method for assembling the connection system 1 as described above.

These three steps can be performed in any order. For example, the assembly of the catheter can be performed before the fluidic connection to the two-lumen catheter or the fluid management device.

Thanks to the same structure of the proximal part of the embodiments of the fluid management device with two device connectors or a single device connector, it is possible to easily replace the fluid management device with two device connectors by the fluid management device with a single device connector at the end of the dialysis for example to inject the lock solution.

## Claims

1. A connection system (1) for connecting a two-lumen catheter to a fluid management device, the connection system (1) comprising a catheter connecting device (10) and a management connecting device (20, 40) configured to be connected together,
wherein the catheter connecting device (10) comprises:
- a housing comprising
∘ a first catheter connector (11a) and a second catheter connector (11b), each catheter connector (11a, 11b) being configured to be fluidically connected to a lumen of a two-lumen catheter;
∘ a connection aperture (15c) opposed to the two catheter connectors (11a, 11b);
∘ an external surface (15d) extending from the catheter connectors (11a, 11b) to the connection aperture (15c), the catheter connectors (11a, 11b) and the connection aperture (15c) defining a flow axis (A);
- a polymeric gasket (13) disposed within the housing, the gasket (13) comprising a proximal gasket surface (13a) and a distal gasket surface (13b) perpendicular to the flow axis (A), the gasket (13) comprising two through holes (13c) aligned with the catheter connectors (11a, 11b), each through hole (13c) being in fluidic communication with one of the catheter connectors (11a, 11b);
- a rotor (14) disposed within the housing and abutted against the distal gasket surface (13b), the rotor (14) comprising an opening (14a) configured to allow fluidic communication from the catheter connectors (11a, 11b) towards the connection aperture (15c); and
wherein the management connecting device (20, 40) comprises:
- a locking element configured to reversibly lock the management connecting device (20, 40) to the catheter connecting device (10) thereby preventing relative movement between the management connecting device (20, 40) and the catheter connecting device (10) along the flow axis (A);
- a socket (21, 41) comprising a first device connector (24a, 44) configured to be fluidically connected to a fluid management device;
the socket (21, 41) being configured to allow fluidic communication between the first device connector (24a, 44) and the opening (14a) of the rotor (14), when the management connecting device (20, 40) and the catheter connecting device (10) are connected together through the connection aperture (15c);
the management connecting device (20, 40) being configured, when the management connecting device (20, 40) and the catheter connecting device (10) are connected, to switch between a forward configuration wherein the first catheter connector (11a) is in fluidic communication with the first device connector (24a, 44) and a reverse configuration wherein the second catheter connector (11b) is in fluidic communication with the first device connector (24a, 44) by rotation of the socket (21, 41) around the flow axis (A).

2. The connection system (1) according to claim 1, wherein the socket (21) of the management connecting device (20) comprises a second device connector (24b) configured to be fluidically connected to a fluid management device; the socket (21) being configured to allow fluidic communication between the second device connector (24b) and the opening (14a) of the rotor (14), when the management connecting device (20) and the catheter connecting device (10) are connected together through the connection aperture (15c); wherein, in the forward configuration, the second catheter connector (11b) is in fluidic communication with the second device connector (24b) and, in the reverse configuration, the first catheter connector (11a) is in fluidic communication with the second device connector (24b).

3. The connection system (1) according to claim 1 or 2, wherein the polymeric gasket (13) comprises silicone, thermoplastic elastomer, ethylene propylene diene monomer rubber, or a mixture thereof.

4. The connection system (1) according to any one of claims 1 to 3, wherein the catheter connecting device (10) further comprises a gasket support (12) disposed within the housing, the gasket support (12) comprising a proximal support surface (12a) and a distal support surface (12b) perpendicular to the flow axis (A), the gasket support (12) comprising two support channels (12c), each support channel (12c) being in fluidic communication with one of the catheter connectors (11a, 11b), the proximal gasket surface (13a) being abutted against the distal support surface (12b), each through hole (13c) being fluidically connected with one of the support channels (12c).

5. The connection system (1) according to any one of claims 1 to 4, wherein the socket (21, 41) comprises at least one rotational key (21b, 41b), the at least one rotational key (21b, 41) comprising at least one socket channel (21c, 41c) aligned with the device connector (24a, 24b, 44) and being configured to cooperate, when the management connecting device (20, 40) and the catheter connecting device (10) are connected, with the opening (14a) of the rotor (14) thereby providing rotational locking between the rotor (14) and the socket (21, 41).

6. The connection system (1) according to any one of claims 1 to 5, wherein the housing has a general cylindrical shape, the external surface (15d) of the housing comprises an annular groove (15a) and the locking element comprises at least one socket pin (21a, 41a) fixed to the socket (21, 41) and configured to slid along the annular groove (15a) when the management connecting device (20, 40) and the catheter connecting device (10) are connected thereby allowing rotation of the socket (21, 41) around the flow axis (A).

7. The connection system according to any one of claims 1 to 6, wherein the external surface (15d) of the housing comprises a L-shaped groove (15b), the L-shaped groove (15b) comprising an insertion part extending parallelly to the flow axis (A) from the connection aperture (15c) and a locking part extending perpendicularly to the flow axis (A) from the extremity of the insertion part, the management connecting device (20, 40) further comprising a locking ring (22, 42) configured to surround at least part of the socket (21, 41), the locking element comprising at least one locking pin (22a, 42a) protruding inside the locking ring (22, 42), the at least one locking pin (22a, 42a) being configured to slide along the L-shaped groove (15b) to lock the management connecting device (20, 40) and the catheter connecting device (10) when the at least one locking pin (22a, 42a) is in the locking part of the L-shaped groove (15b).

8. The connection system (1) according to claim 7 depending on claim 6, wherein the annular groove (15a) crosses the insertion part of the L-shaped groove (15b).

9. The connection system (1) according to any one of claims 1 to 8, wherein the catheter connecting device (10) further comprises a cap (30) configured to seal the connection aperture (15c) when the management connecting device (20, 40) and the catheter connecting device (10) are not connected.

10. The connection system (1) according to claim 9, wherein the cap (30) comprises at least one cap key, the at least one cap key being configured to cooperate, when the cap (30) seals the connection aperture (15c), with the opening (14a) of the rotor (14) thereby preventing rotation of the rotor (14).

11. The connection system (1) according to any one of claims 1 to 10, wherein the catheter connectors (11a, 11b) are luer-locks.

12. The connection system (1) according to any one of claim 11, wherein the catheter connectors (11a, 11b) comprise a plurality of teeth (11c) cooperating with a pin (12d) fixed on the housing of the catheter connecting device (10) to prevent rotation between the catheter connectors (11a, 11b) and the housing.

13. The connection system (1) according to any one of claims 1 to 12, wherein the catheter connecting device (10) comprises biocompatible material.

14. The connection system (1) according to any one of claims 1 to 13, wherein the two-lumen catheter is a central venous catheter.

15. The connection system (1) according to claim 14, wherein the fluid management device is a dialysis device or a lock solution injector.
